(19) Europäisches Patentamt
European Patent Office
Office européen des brevets

(11) Publication number: 0 096 974 B1

(12) # EUROPEAN PATENT SPECIFICATION

(45) Date of publication of the patent specification: 11.09.85

(51) Int. Cl.⁴: C 07 C 51/54, C 07 C 53/12, C 07 C 53/122

(21) Application number: 83302846.7

(22) Date of filing: 19.05.83

(54) **Process for the production of one or more carboxylic acid anhydrides.**

(30) Priority: 22.05.82 GB 8215013

(43) Date of publication of application: 28.12.83 Bulletin 83/52

(45) Publication of the grant of the patent: 11.09.85 Bulletin 85/37

(84) Designated Contracting States: BE DE FR GB IT NL

(56) References cited:
US - A - 2 689 261
US - A - 3 040 090
US - A - 3 168 553
US - A - 3 857 900

(73) Proprietor: BP Chemicals Limited, Belgrave House 76 Buckingham Palace Road, London, SW1W 0SU (GB)

(72) Inventor: Ray, David John Mathieson, BP Chemicals Limited Salt End, Hedon Hull HU12 8DS (GB)
Inventor: Williams, Peter Sefton, BP Chemicals Limited Salt End, Hedon Hull, HU12 8DS (GB)

(74) Representative: Crack, Richard David et al, c/o The British Petroleum Company plc Patents Division Chertsey Road, Sunbury-on-Thames Middlesex, TW16 7LN (GB)

EP 0 096 974 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

BUNDESDRUCKEREI BERLIN

**Description**

The present invention relates to a process for the production of one or more carboxylic acid anhydrides.

Acetic anhydride has been known as an industrial chemical for many years. It constitutes the second largest end use for acetic acid and is extensively employed in the production of cellulose acetate and other esters. Smaller quantities are used in the production of aspirin, pharmaceuticals, pesticides and low temperature bleach activators. Uses have been proposed for other carboxylic acid anhydrides, such as propionic anhydride.

On an industrial scale acetic anhydride is currently produced either by reaction of ketene and acetic acid, the ketene being obtained either by dehydration of acetic acid or by decomposition of acetone, or by the oxidation of acetaldehyde, which also yields acetic acid. Each of these conventional routes has well-known disadvantages which knowledge, together with the increasing attention being paid to chemical syntheses involving carbon monoxide and carbon monoxide/hydrogen mixtures, has led to investigations into the production of acetic anhydride utilising these materials. One approach to the preparation of an anhydride of a monocarboxylic acid is described in the complete specification of GB 1 468 940, in which a carboxylate ester satisfying the formula RCOOR or an ether satisfying the formula ROR is reacted with an acyl halide satisfying the formula RCOX, formed in situ or in a separate stage, under substantially anhydrous conditions, wherein X is iodide or bromide, the Rs may be the same or different and each R is a monovalent hydrocarbon radical or a substituted monovalent hydrocarbon radical wherein the or each substituent is inert. The acyl halide may be produced by carbonylation of a halide satisfying the formula RX at superatmospheric pressure in the presence as catalyst of a Group VIII noble metal. Another approach to the production of carboxylic acid anhydrides is described in the complete specifications of GB Patents Nos 1 398 530, 1 367 607 and 1 448 010 (all to Monsanto Co) in which an ethylenically unsaturated compound is reacted with carbon monoxide in the presence of a carboxylic acid and a catalyst which is either a Group VIII metal or metal compound and a phenol, thiophenol, a substituted phenol or thiophenol, a $C_{1-30}$ alkanethiocarboxylic acid, a $C_{1-30}$ fluoroalkanecarboxylic acid, a $C_{1-30}$ fluoroalkanethiocarboxylic acid or a $C_{1-30}$ fluoroalkanesulphonic acid as promoter (GB 1 398 530), or an iridium complex comprising at least one ligand derived from (a) a compound which is a tertiary organoderivative of phosphorus, arsenic or antimony in which the total number of carbon atoms in the organic groups is from 3 to 90, (b) an olefinic compound having from 2 to 40 carbon atoms, or (c) a diolefinic compound having from 4 to 40 carbon atoms, in the substantial absence of added halide componentes (GB 1 367 607) or (a) an unchelated rhodium (I), rhodium (III), iridium (I) or iridium (III) component, (b) an iodide component, and (c) a catalyst preserver or regenerator component which comprises hydrogen or a compound which yields hydrogen under the reaction conditions (GB 1 448 010). Replacement of the carboxylic acid by water at a quantitative level at least stoichiometric with the olefin reactant results in the formation of carboxylic acids rather than their anhydrides.

US 3 168 533 assigned to Shell Oil Company discloses a catalytic process for the production of carbonyl compounds including acid anhydrides from olefins, carbon monoxide and hydrogen containing compounds such as alcohols. The catalyst used therein consists essentially of cobalt, ruthenium, rhodium, or iridium in combination with carbon monoxide and a tri-alkylphosphine.

It has now been found that a carboxylic acid anhydride or a mixture of carboxylic acid anhydrides can be produced by reacting an olefinically unsaturated compound and an alcohol with carbon monoxide in the presence of rhodium or iridium as catalyst and a halogen promoter.

Accordingly, the present invention provides a process for the production of a carboxylic acid anhydride or a mixture of carboxylic acid anhydrides comprising reacting an olefin and an alcohol with carbon monoxide at elevated temperatures, the process characterised in that the reaction occurs in the presence of a catalyst containing rhodium or iridium and a promoter comprising a halogen selected from bromine and iodine in free or combined form.

The olefin may suitably be selected from compounds having from 2 to 30 carbon atoms of formula:

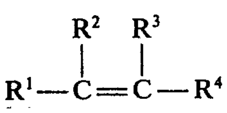

(I)

in aliphatic, heteroaliphatic, acyclic or cycloaliphatic form wherein $R^1$, $R^2$, $R^3$ and $R^4$ are independently either hydrogen, halogen, alkyl, alkenyl, aryl, cycloalkyl or cycloalkenyl moieties or, in a heteroaliphatic compound, are moieties containing nitrogen, phosphorus, sulphur, halogen or oxygen atoms or, in a cycloaliphatic compound $R^2$ and $R^3$ are linked. Suitable compounds having the above formula (I) include ethylene; propylene; butene-1; butene-2; pentenes; hexenes; octenes; hexadecene; 2-methylpropene; 1,3-butadiene; 2-methyl-1,3-butadiene; 2,3-dimethyl-1,3-butadiene; styrene; methylstyrene; 3,3-dimethyl-1-butene; 1,4-hexadiene; acrolein; methyl vinyl ketone and 2-cyclohexylbutene. If desired, mixtures of the aforesaid olefins may be employed.

The alcohol may be an aliphatic, heteroaliphatic, acyclic, cycloaliphatic or aromatic alcohol, or a mixture thereof. Suitably the alcohol may be an alkanol having from 1 to 10 carbon atoms, such as

methanol, ethanol, n-propanol, iso-propanol, n-butanol, sec-butanol or tert-butanol.

The carbon monoxide may suitably be substantially pure or it may contain impurities such as hydrogen, carbon dioxide, nitrogen or methane.

If water is present in the reactants the yield of anhydride product is reduced and carboxylic acid is produced. Preferably, therefore, in order to reduce the formation of substantial amounts of carboxylic acids all the reactants including the carbon monoxide are substantially anhydrous, for example they contain less than 3%, more preferably less than 1% w/w water.

The catalyst comprises rhodium or iridium. The rhodium or iridium may be used in the zero valent state or in any higher valent form. Thus the rhodium or iridium may be added as the elemental metal in finely divided form, as a simple salt such as the halide, as an organometallic compound such as a carboxylate salt or as a coordination compound with ligands such as carbon monoxide, halides, phosphines, arsines and trivalent nitrogen compounds. Suitable forms of rhodium or iridium are described in the patent specifications of GB 1 253 758 and the aforesaid GB 1 398 530, 1 367 607 and 1 448 010 to which reference may be made for further details. Examples of compounds which may be added include $[Rh(CO)_2Cl]_2$; $RhCl_3 \cdot 2H_2O$; $RhBr_3$; $RhI_3$; $IrBr_3$; $IrI_3$; $RhBr_3 \cdot 3H_2O$; $IrBr_3 \cdot 3H_2O$; $Rh_2(CO)_4Br_2$; $Ir_2(CO)_4I_2$; $Rh[(C_6H_5)_3P]_2(CO)I$ and $IrBr[(C_6H_5)_3P]_3$.

Suitably the promoter may be added in the from of elementary iodine, hydrogen iodide, an inorganic halide salt, such as for example sodium or potassium iodide, or a quaternary ammonium or phosphonium halide, such as for example a tetraalkylammonium halide. Particularly preferred are organo-iodides and bromides such as alkyl iodides, eg methyl iodide.

Preferably a quaternary nitrogen, phosphorus or arsenic copromoter is present. The copromoter can be a heterocyclic aromatic compound in which at least one of the hetero atoms is a quaternary nitrogen atom. The copromoter can be added as such or in a from capable of forming the quaternary compound under the reaction conditions. Suitable heterocyclic aromatic quaternary nitrogen compounds which may be added as such include N-methyl pyridinium iodide; N,N-dimethylimidazolinium iodide; N-methyl-3-picolinium iodide; N-methyl-3, 4-lutidinium iodide and N-methyl-quinolinium iodide. Suitable quaternary phosphorus compounds include tributylmethylphosphonium iodide, triphenylmethylphosphonium iodide, trilaurylmethylphosphonium iodide and trioctylmethylphosphonium iodide. It is preferred, however, to add a heterocyclic aromatic compound of trivalent nitrogen which is capable of forming a quaternary compound under the reaction conditions. Suitable trivalent forms of nitrogen are heterocyclic amines such as pyridine, picoline, quinoline, methylquinoline, hydroxyquinoline pyrrole, pyrrolidine, pyrrolidone and the like or an imidazole, such as imidazole, methyl imidazole and the like. Alternatively, there may be added a compound of formula:

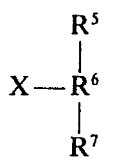

(II)

wherein X is nitrogen, phosphorus or arsenic and $R^5$, $R^6$ and $R^7$ which may be the same or different are alkyl groups having up to 20, preferably from 1 to 8, carbon atoms or monocyclic aryl groups, or when X is phosphorus or arsenic $R^7$ may be the group:

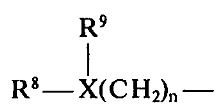

wherein $R^7$ and $R^8$ are each a monocyclic aryl group or an alkyl group and n is zero or an integer in the range 1 to 20. Suitable compounds of formula (II) include trimethylamine, triethylamine, triphenylamine, triphenylarsine, methyldiphenylarsine, trimethylphosphine, tripropylphosphine and triphenylphosphine.

With regard to the amounts of the various reactants employed, the molar ratio of olefin to methanol to carbon monoxide may suitably be from 0.1 to 10: 0.1 to 10: 0.1 to 10 in particular from 0.75 to 1.5: 0.75 to 1.5: 0.75 to 1.5 for example about 1 : 1 : 1, though different ratios may be employed if so desired. Although any ratio of promoter to rhodium or iridium may be employed, suitable ratios expressed as atoms of iodine or bromide in the promoter to atoms of rhodium or iridium in the catalyst may suitably be in the range from 1 : 1 to 2500 : 1, preferably at least 3 : 1 for example from from 3 : 1 to 300 : 1. Using a copromoter which is formed 'in situ' it will be necessary to make adjustments to the foregoing ranges to make allowance for the fact that iodine is utilised in forming the quaternary compound. The molar ratio of rhodium or iridium compound in the catalyst component to the quaternary compound employed as copromoter may suitably be not more than 1 : 1 for example in the range from 1 : 1 to 1 : 1200, preferably from 1 : 10 to 1 : 300.

The elevated temperature may suitably be in the range from 50 to 300°C, preferably from 125 to 250°C. The partial pressure of carbon monoxide and olefin (when the olefin is gaseous) may sutably be in the range from 1.0 to 1000 bar, preferably from 10 to 100 bar. Higher pressures may be used if so desired under appropriate conditions.

The process may be carried out in the liquid phase or in the vapour phase. In the case of both liquid and vapour phase operation, the catalyst and optionally also the promoter or copromoter may be supported, ie they may be dispersed on a conventional support material, such as for example alumina, silica, silica/alumina, zeolites, clays, titania or zirconia. The catalyst and optionally the promoter and/or copromoter may be applied to the support in conventional manner, eg by impregnation from solution. The catalyst concentration on the support may suitably be in the range from 0.01 to 10% by weight. Alternatively, the metal catalyst components may be attached to an ion exchange resin or to a functionalised inorganic oxide such as those described in the complete specification of GB 1 503 315 or European patent publication No 18102 (BP Case No 4752).

The process of the invention may be carried out batchwise or continuously.

Reaction of methanol and ethylene with carbon monoxide is believed to proceed according to the following equations:

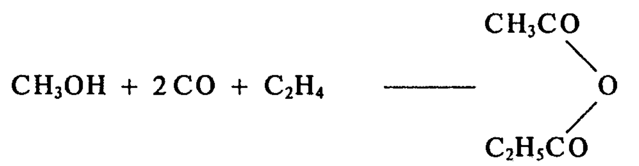

(I)

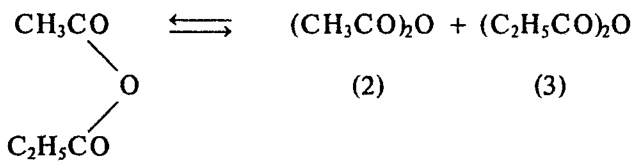

(2) (3)

(1)

The product contains a mixture of three acid anhydrides (1), (2) and (3). This mixture may be isolated from the product, suitably by distillation, and utilised without any further processing or may be separated into its individual components by distillation. Alternatively, the mixture of products (1), (2) and (3) may be distilled in the presence of a catalyst for accelerating the interconversion of (1) into (2) and (3) and acetic anhydride (2) distilled out of the mixture, thereby driving the equilibrium in the direction of (2) and (3) and recovering two instead of three acid anhydrides. Reaction of ethanol and propylene with carbon monoxide gives a mixture of propionyl anhydride, butyryl anhydride and the mixed propionyl butyryl anhydride. It is possible to produce a single carboxylic acid anhydride product. Thus, for example, reaction of ethanol and ethylene with carbon monoxide can produce only propionic anhydride. Thus in general reaction of a $C_{n-1}$ alcohol and a $C_{n-1}$ olefin with carbon monoxide produces a single anhydride $(C_{n-1}CO)_2O$.

The invention will now be further illustrated by reference to the following Examples.

## Example 1

### No quaternary nitrogen copromoter present

A corrosion-resistant autoclave of 100 $cm^3$ capacity equipped with a rotary stirrer was charged with the following reaction mixture: methanol 16 g, methyl iodide 2.8 g, and $[Rh(CO)_2Cl]_2$ 0.1 g. The autoclave was closed, flushed three times with ethylene and then pressurised with 500 psia of ethylene and 500 psia of carbon monoxide. The autoclave was heated to 170°C and this temperature maintained for 10 hours. The autoclave was then cooled and depressurised and a sample withdrawn for analysis. The autoclave was then repressurised with 500 psia of ethylene and 500 psia of carbon monoxide and reheated to 170°C. 14 hours later the autoclave was cooled and depressurised. Gas liquid chromatography (GLC) analysis showed that after 10 hours the product contained no anhydrides but that after 24 hours anhydrides were formed in the following concentrations: acetic anhydride 1%, aceto-propionic anhydride 8.6% and propionic anhydride 14.2% w/w.

Example 2

Use of quaternary nitrogen copromoter

To the autoclave of Example 1 was charged methanol 16 g, methyl iodide 2.8 g, $[Rh(CO)_2Cl]_2$ 0.1 g and methyl 3-picolinium iodide (quaternary nitrogen copromoter) 9.4 g. The autoclave was flushed three times with ethylene and then pressurised with 470 psia of ethylene and 520 psia of carbon monoxide. The autoclave was heated to 170°C and this temperature maintained for 3 hours, during which time 880 psia of carbon monoxide was added to replace gas consumed by the reaction. The autoclave was then cooled and depressurised. GLC analysis showed that the product contained anhydrides in the following concentrations: acetic anhydride 8.4%, aceto-propionic anhydride 8.2% and propionic anhydride 2.7 w/w.

Example 3

Use of quaternary nitrogen copromoter formed in situ

The autoclave of Example 1 was charged with a reaction mixture comprising methanol 16 g, methyl iodide 10.9 g, N-methyl imidazole 1.78 g, and $RhCl_3 \cdot 3H_2O$ 0.1 g. The autoclave was closed, flushed three times with ethylene, and then pressurised with 520 psia ethylene and 475 psia CO. The autoclave was heated and the temperature maintained at 170°C for 3.5 hours, during which time the pressure was maintained at greater than 650 psia by addition of fresh CO to replace gas consumed by the reaction. The product was analysed by GLC and found to contain anhydrides in the following concentrations: acetic anhydride 14.8%, aceto propionic anhydride 13.4%, and propionic anhydride 3.6% w/w.

In all the above examples the alcohol was maintained in the liquid phase and the catalyst in solution.

**Claims**

1. A process for the production of a carboxylic acid anhydride or a mixture of carboxylic acid anhydrides comprising reacting an olefin and an alcohol with carbon monoxide at elevated temperatures, the process characterised in that the reaction occurs in the presence of a catalyst rhodium or iridium and a promoter comprising a halogen slected from iodine and bromine in free or combined form.

2. A process as claimed in claim 1 wherein a quaternary nitrogen, phosphorus or arsenic copromoter is present.

3. A process as claimed in claim 2 wherein the quaternary nitrogen phosphorus or arsenic copromoter is formed by adding to the reaction vessel a trivalent nitrogen, phosphorus or arsenic compound capable of forming the quaternary compound under the reaction conditions.

4. A process as claimed in claim 3 wherein the trivalent nitrogen compound is one containing the trivalent nitrogen in a heterocycle.

5. A process as claimed in any one of claims 1 to 4 wherein the atomic ratio of halogen to rhodium or iridium is at least 3 : 1.

6. A process as claimed in any one of claims 2 to 5 wherein the ratio of atoms of rhodium or iridium to molecules of copromoter is not more than 1 : 1 preferably 1 : 10 to 1 : 300.

7. A process as claimed in any one of the preceding claims wherein the process is effected at a temperture in the range 50 to 300°C and a partial pressure of carbon monoxide of from 10 to 100 bar and a partial pressure of olefin from 10 to 100 bar under conditions to maintain the alcohol or ester in the liquid phase and the catalyst in solution.

**Patentansprüche**

1. Verfahren zur Herstellung eines Carbonsäureanhydrids oder einer Mischung von Carbonsäureanhydriden durch Umsetzung eines Olefins und eines Alkohols mit Kohlenmonoxid bei erhöhten Temperaturen, dadurch gekennzeichnet, daß die Umsetzung in Anwesenheit eines Rhodium oder Iridium enthaltenden Katalysators und eines Promotors durchgeführt wird, der ein Halogen ausgewählt unter Jod und Brom in freier oder gebundener Form enthält.

2. Verfahren gemäß Anspruch 1, worin ein quaternären Stickstoff, Phosphor oder Arsen enthaltender Copromotor anwesend ist.

3. Verfahren gemäß Anspruch 2, worin der quaternären Stickstoff, Phosphor oder Arsen enthaltende Copromotor gebildet wird, indem in das Reaktionsgefäß eine Verbindung mit dreiwertigem Stickstoff, Phosphor oder Arsen, die in der Lage ist, unter den Reaktionsbedingungen die quaternäre Verbindung zu bilden, zugegeben wird.

4. Verfahren gemäß Anspruch 3, worin die dreiwertigen Stickstoff enthaltende Verbindung eine solche ist, die den dreiwertigen Stickstoff in einem Heterozyklus enthält.

5. Verfahren gemäß einem der Ansprüche 1 bis 4, worin das Atomverhältnis von Halogen zu Rhodium oder Iridium mindestens 3 : 1 beträgt.

6. Verfahren gemäß einem der Ansprüche 2 bis 5, worin das Verhältnis der Atome von Rhodium oder Iridium zu den Molekülen des Copromotors nicht mehr als 1 : 1, vorzugsweise 1 : 10 bis 1 : 300, beträgt.

7. Verfahren gemäß einem der vorgehenden Ansprüche, worin das Verfahren bei einer Temperatur im Bereich von 50 bis 300°C und bei einem Partialdruck des Kohlenmonoxids von 10 bis 100 bar und einem Partialdruck des Olefins von 10 bis 100 bar unter Bedingungen durchgeführt wird, daß der Alkohol oder Ester in der flüssigen Phase und der Katalysator in der Lösung bleiben.

**Revendications**

1. Procédé de production d'un anhydride d'acide carboxylique, ou d'un mélange d'anhydrides d'acides carboxyliques, comprenant la réaction d'une oléfine et d'un alcool avec le monoxyde de carbone à des températures élevées, procédé caractérisé en ce que la réaction se produit en présence d'un catalyseur contenant du rhodium ou de l'iridium et d'un promoteur comprenant un halogène choisi parmi l'iode et le brome sous forme libre ou combinée.

2. Procédé selon la revendication 1, dans lequel un copromoteur contenant de l'azote, du phosphore ou de l'arsenic quaternaires est présent.

3. Procédé selon la revendication 2, dans lequel le copromoteur, à azote, phospore ou arsenic quaternaires est formé par introduction, dans le récipient de réaction, d'un composé à azote, phosphore ou arsenic trivalents, capable de former le composé quaternaire dans les conditions de réaction.

4. Procédé selon la revendication 3, dans lequel le composé à azote trivalent est un composé contenant l'azote trivalent dans un hétérocycle.

5. Procédé selon l'une quelconque des revendications 1 à 4, dans lequel le rapport atomique de l'halogène au rhodium ou à l'iridium est au moins égal à 3 : 1.

6, Procédé selon l'une quelconque des revendications 2 à 5, dans lequel le rapport des atomes de rhodium ou d'iridium aux molécules du promoteur n'est pas supérieur à 1 : 1 et se situe de préférence entre 1 : 10 et 1 : 300.

7. Procédé selon l'une quelconque des revendications précédentes, dans lequel on met le procédé en oeuvre à une température comprise entre 50 et 300°C et à une pression partielle du monoxyde de carbone de 10 à 100 bars et à une pression partielle de l'oléfine de 10 à 100 bars, dans des conditions destinées à maintenir l'alcool ou l'ester en phase liquide et le catalyseur en solution.